(19) Europäisches Patentamt
European Patent Office
Office européen des brevets

(11) Publication number: 0 309 248 A2

(12) # EUROPEAN PATENT APPLICATION

(21) Application number: 88308810.6

(22) Date of filing: 22.09.88

(51) Int. Cl.4: C 07 C 69/54
C 07 C 67/297, C 07 D 319/06

(30) Priority: 22.09.87 US 99555

(43) Date of publication of application:
29.03.89 Bulletin 89/13

(84) Designated Contracting States:
DE ES FR GB IT SE

(71) Applicant: MINNESOTA MINING AND MANUFACTURING COMPANY
3M Center, P.O. Box 33427
St. Paul, Minnesota 55133-3427 (US)

(72) Inventor: Johnson, Gilbert C. c/o Minnesota Mining and Manufacturing Company 2501 Hudson Road
P.O. Box 33427 St. Paul Minnesota 55133 (US)

(74) Representative: Baillie, Iain Cameron et al
*c/o Ladas & Parry Isartorplatz 5*
D-8000 München 2 (DE)

(54) **Acrylated diols.**

(57) Polymerizable esters of 2,2-bis(hydroxymethyl)-1,3-propanediol with acrylic and methacrylic acids and mixtures of acrylic or methacrylic acid and other organic carboxylic acids are disclosed. Polymers of these esters and mixture of esters are crosslinkable and useful in many applications, particularly in protective coatings.

EP 0 309 248 A2

**Description**

## ACRYLATED DIOLS

FIELD OF THE INVENTION

This invention relates to diols having pendent acrylate or methacrylate groups that are uncontaminated by monools, triols, and polyols. In particular, the invention relates to 2,2-bis(hydroxymethyl)-1,3-propanediol esters of acrylic and methacrylic acid and mixtures of these esters with 2,2-bis(hydroxymethyl)-1,3-propanediol esters of other organic carboxylic acids that are not contaminated by monools, triols and higher polyols. The invention further relates to a process for preparing the uncontaminated esters. In another aspect the invention relates to condensation polymers prepared therefrom.

DESCRIPTION OF THE PRIOR ART

Acrylic acid ester substituted diols are known. U.S. Patent Nos. 3,210,327 and 3,267,084 disclose monoacrylic and monomethacrylic acid esters of alkanetriols having the formula

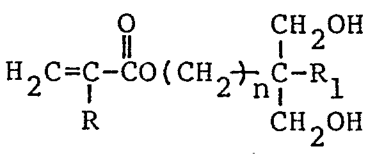

wherein R is hydrogen or lower alkyl group, $R_1$ is lower alkyl group having 1 to 4 carbon atoms or hydrogen, and n is an integer of 1 to 4, inclusive. These esters are prepared by the hydrolysis of the corresponding ketal. U.S. Patent Nos. 4,366,301 and 4,367,302 disclose acrylic and methacrylic acid esters of trihydric alcohols of the formula

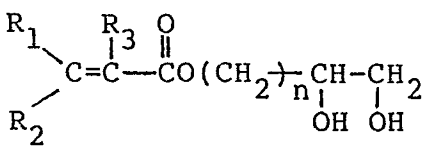

in which $R_1$ and $R_2$ are independently hydrogen, lower alkyl group, aryl groups, or halogen, $R_3$ is hydrogen, lower alky, aryl or cycloalkyl group or halogen, and n is an integer between 1 and 4. These materials have secondary hydroxyl groups which are often sluggish to react in condensation polymerization reactions.

U.S. Patent No. 4,405,798, 4,529,786 and 4,526,949 disclose acrylic and methacrylic acid monoester of pentaerythritol that are prepared by the hydrolysis of an acrylic or methacrylic ester of pentaerythritol orthoester in accordance with the equation

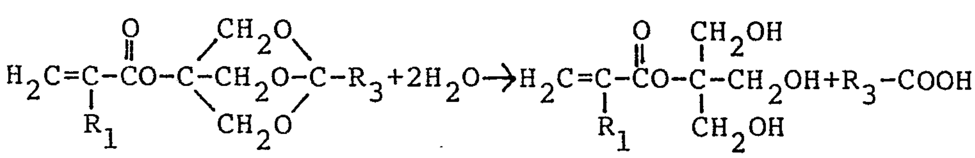

in which $R_1$ is hydrogen or methyl and $R_3$ is hydrogen or a non-functional organic group. Although these monoacrylic esters can be produced uncontaminated by di and triacrylate or methacrylate esters, neither the pentaeryethritol diacrylate nor the dimethacrylate uncontaminated by mono and triacrylate or methacrylate ester is disclosed. Furthermore the trihydroxyl compounds disclosed in U.S. Patent 4,405,798 are not useful for making linear condensation polymers.

Unsaturated polyesters that are the reaction product of polyepoxides and acrylic or methacrylic acid are known. These compounds contain at least two each of acrylic and methacrylic acid groups and hydroxyl groups that are produced, typically, by the reaction, for example:

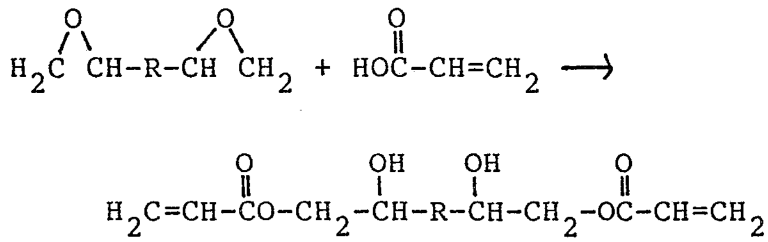

in which R is a divalent group such as, for example,

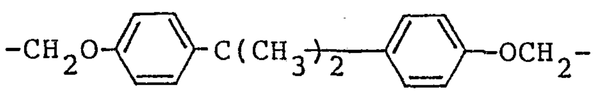

Unsaturated polyesters of this type are disclosed, for example in U.S. Patent Nos. 3,373,221; 3,966,681; 4,162,274; 4,295,947; and 4,334,034. Compounds of this type, even though they contain 2 or more of acrylic and methacrylic groups and 2 or more of hydroxyl groups, have their hydroxyl groups located on secondary carbon atoms and are therefore less desirable in condensation products than the compounds of the invention which have their hydroxyl groups located on primary carbon atoms. Furthermore, these materials introduce a significant amount of residue from the body of the diepoxide into the condensation product.

U.S. Patent No. 4,216,019 discloses photostencils utilizing compositions comprising ethylenically unsaturated monomers and polymers, particularly acrylic acid esters of mono, di, tri, and tetrahydric alcohols. It is stated at col. 4, line 65, to col. 5, line 9, that, "Acrylate esters of...Monohydric alcohols...Dihydric alcohols...Trihydric alcohols...Tetrahydric alcohols: pentaerythritol...Polyhydric alcohols: are suitable for use in the composition. All hydroxyl groups may be esterified or one or more groups may be left unesterified..." Such a broad description of acrylate esters does include compositions that would contain compounds containing two acrylate groups and two hydroxyl groups, however, there is no teaching or suggestion of compositions that contain pentaerythritol diacrylate that would not be contaminated by pentaerythritol monoacrylate having three hydroxyl groups and pentaerythritol triacrylate having one hydroxyl groups. It is taught in U.S. Patent No. 4,216,019 that these acrylic acid esters are prepared "for example by reacting acrylic acid or acryloyl chloride with a polymer or polymerizable material containing hydroxyl groups". It is known that when the hydroxyl group-containing material is pentaerythritol such a reaction cannot give an uncontaminated pentaerythritol diacrylate but does give a mixture of mono, di, tri and tetracylates having three, two, one and no hydroxyl groups [See Japan Kokai 77,113,916 (Chem. Abs. 88, P50312r (1978)] where it is taught that esterification of pentaerythritol with acrylic acid gives a mixture of a trace of monoacrylate, 7% diacrylate, 54% triacrylate, and 34% tetraacrylate].

SUMMARY OF THE INVENTION

Briefly, in accordance with the present invention there is provided 2,2-bis(hydroxymethyl)-1,3-propanediol esters of acrylic and methacrylic acids that are uncontaminated by monools, triols, and higher polyol-containing oligomers of pentaerythritol.

Preferably, the diol esters are represented by the formula:

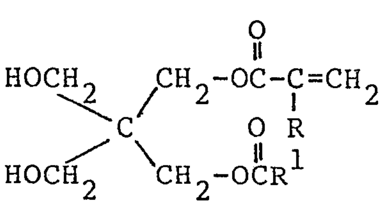
I

wherein

R is hydrogen or methyl, and

$R^1$ is a saturated or unsaturated aliphatic linear, branched, or cyclic group having 1 to 24 carbon atoms optionally containing groups which are unreactive in the esterification process, such as chlorine, fluorine atoms, or ether oxygen.

The invention also provides a process for the preparation of 2,2-bis(hydroxymethyl)-1,3-propanediol esters

of acrylic or methacrylic acid or mixtures of these esters with 2,2-bis(hydroxymethyl)-1,3-propanediol esters of other saturated and unsaturated aliphatic and aromatic monocarboxylic acids, the esters and mixtures of esters being uncontaminated by monools, triols, and higher polyol-containing oligomers of pentaerythritol and are represented by the general formula:

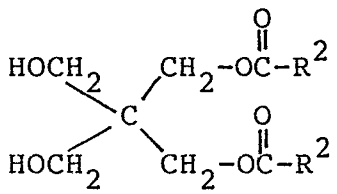

II

wherein each $R^2$ is the same or different monovalent saturated or unsaturated aliphatic linear, branched, or cyclic group having 1 to 24 carbon atoms, or aromatic groups having 6 to 18 carbon atoms, optionally containing groups and atoms unreactive in the esterification reaction such as chlorine, fluorine, or ether oxygen with the proviso that at least, 1 mole percent, preferably 5 mole percent, by weight of the $R^2$ groups is $-\underset{R}{\underset{|}{C}}=CH_2$,

in which R is hydrogen or methyl, comprising the steps of:

a) esterifying a 2-mono or 2,2-diorganic group-substituted 5,5-bis(hydroxymethyl)-1,3-dioxane represented by the formula:

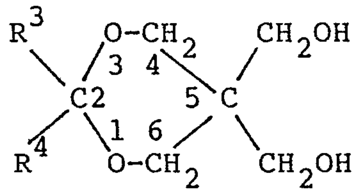

III

wherein

$R^3$ is a lower alkyl group of 1 to 6 carbon atoms or a phenyl group, optionally substituted by a non-interfering group or atom, as for example, alkoxy of 1 to 4 carbon atoms, dialkylamino wherein alkyl has 1 to 4 carbon atoms, alkyl group of 1 to 4 carbon atoms or halogen, and

$R^4$ is independently the same or different $R^3$ or hydrogen,

with two equivalents of at least one aliphatic carbonyl compound represented by the formula:

$R^2\text{-}\overset{O}{\overset{||}{C}}\ X$ IV

wherein

$R^2$ is defined above and

X is hydroxyl, halogen, alkoxy having 1 to 4 carbon atoms or $-O\overset{O}{\overset{||}{C}}R^2$, wherein $R^2$ is as defined above, to provide one of or a mixture of 2-mono or 2,2-diorganic group substituted 5,5-bis(hydroxymethyl)-1,3-dioxane esters of aliphatic carboxylic acids, $R^2COOH$, where $R^2$ is defined above, the esters having the general formula:

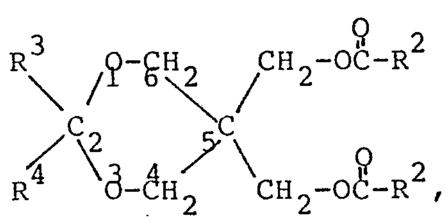

V

wherein $R^2$, $R^3$, and $R^4$ are as defined above;

b) hydrolyzing the dioxane ester or mixture of esters provided in step a) to provide said esters or mixture of said esters of formula V uncontaminated by monools, triols, and higher polyol-containing

oligomers of pentaerythritol.

The invention further provides solvent-soluble, crosslinkable condensation polymers (polyesters and polyurethanes) having pendent acrylic or methacrylic acid ester groups and optionally other pendent aliphatic carboxylic acid ester groups represented by the general formula:

$$W\text{-}(OR^5\text{-}O\overset{O}{\overset{\|}{C}}Y\text{-}R^6\text{-}Y\overset{O}{\overset{\|}{C}}\text{-})_a Z \qquad VI$$

wherein

W and Z are terminal groups, $R^5$ is one or a mixture of two or more divalent groups selected from the residues (provided by removal of two hydroxy hydrogen atoms) from polyetherdiols, polyesterdiols, polyurethanediols, polyolefindiols, and polysiloxanediols, divalent aliphatic linear, branched, and cyclic aliphatic groups having 2 tc 12 carbon atoms and aryl and alkaryl groups having 6 to 15 carbon atoms, of which divalent groups at least about 1 to 100 mole percent are groups represented by the formula:

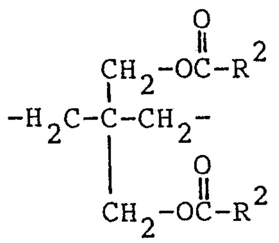

wherein each $R^2$ is defined above;

$R^6$ is one or a mixture of two or more divalent organic groups that is the residue provided by removal of the carboxyl groups from an organic dicarboxylic acid or by the removal of the -N=C=O groups of an organic diisocyanate and is selected from linear, branched, and cyclic aliphatic groups having 2 to 40 carbon atoms and aromatic groups having 5 to 24 carbon atoms and up to 2 nitrogen atoms;

Y is a covalent bond when $R^6$ is the residue provided by removal of carboxyl groups from an organic dicarboxylic acid and is -NH- when $R^6$ is the residue of an organic diisocyanate, and when Y is a covalent bond then

W is H- or $HO\overset{O}{\overset{\|}{C}}\text{-}Y\text{-}R^6\text{-}Y\overset{O}{\overset{\|}{C}}\text{-}$ and
Z is -OH or $\text{-}O\text{-}R^5\text{-}OH$;

and when Y is -NH then

W is H- or $OCN\text{-}R^6\text{-}Y\overset{O}{\overset{\|}{C}}\text{-}$ and
Z is $\text{-}O\text{-}R^5\text{-}O\overset{O}{\overset{\|}{C}}Y\text{-}R^6\text{-}NCO$ or $\text{-}O\text{-}R^5\text{-}OH$;

a is a number of about 2 to 200 such that the number average molecular weight of the polymer is from about 500 to about 50,000 or more.

The crosslinkable polymers of the invention are useful in many applications because of the high concentration of acrylic or methacrylic groups that can be present. High concentration of these groups enables rapid crosslinking of the polymer under the influence of free radicals. Also, with other ethylenically-unsaturated monomers such as methyl methacrylate and styrene, polymer networks are formed leading to many interesting materials. Applications in which the polymers of the invention are useful include for example, coatings, adhesives, caulking and sealing compositions, casting and molding compositions, impregnating compositions, and binders. The crosslinkable polymers are particularly useful in protective coatings because of their excellent abrasion resistance, heat resistance, and solvent resistance.

Assignee's copending patent application U.S.S.N. (attorney's docket No. F.N. 422384 USA 6A filed the same date as the instant invention discloses and claims crosslinkable polyester and polyurethanes having pendent acrylic or methacrylic ester groups comprising the reaction product of (hydroxymethyl)-1,3-propanediol esters of acrylic or methacrylic acid including the 2,2-bis(hydroxymethyl)-1,3-propanediol esters of acrylic or methacrylic acid with dicarboxylic acid and diisocyanates respectively which are disclosed and claimed in the instant invention.

DETAILED DESCRIPTION OF THE INVENTION

This invention provides novel 2,2-bis(hydroxymethyl)-1,3-propanediol esters of acrylic and methacrylic acid prepared as shown in the Flow Chart below, steps a and b.

FLOW CHART

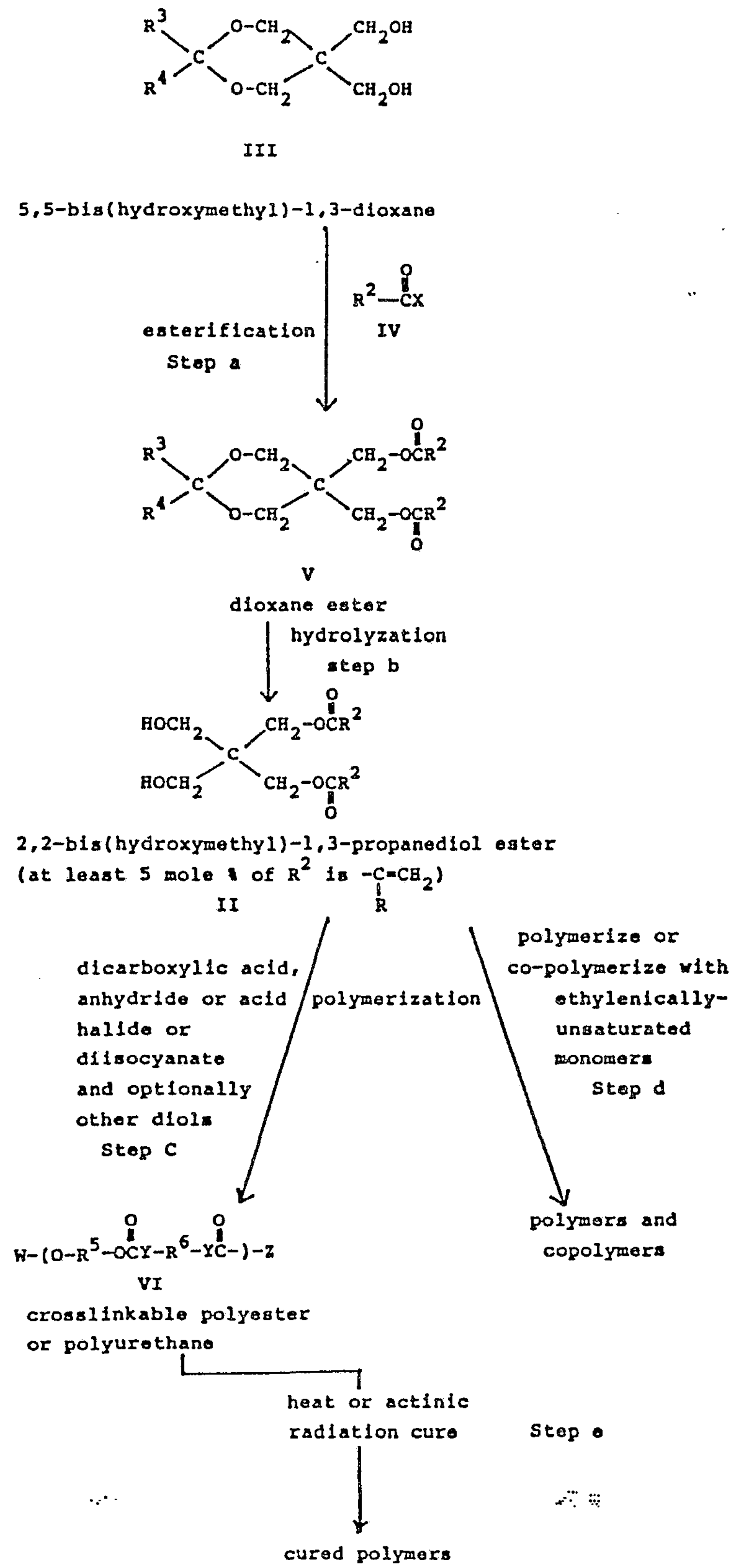


wherein $R^2$, $R^3$, $R^4$, $R^5$, $R^6$, X, W, Z and Y are as previously defined.

The mono and diorganic group-substituted 5,5-bis(hydroxymethyl)-1,3-dioxanes (compounds of Formula III) of use in step 1) of the preparation of the 2,2-bis(hydroxymethyl)-1,3-propanediol esters are known and include, for example, 2,2-dimethyl-5,5-bis(hydroxymethyl)-1,3-dioxane, 2,2-diethyl-5,5-bis(hydroxymethyl)-1,3-dioxane, 2-methyl-2-ethyl-5,5-bis(hydroxymethyl)-1,3-dioxane, 2-phenyl-5,5-bis(hydroxymethyl)-1,2-dioxane, 2-(4-methyoxyphenyl)-5,5-bis(hydroxymethyl)-1,3-dioxane, 2-(4-dimethylaminophenyl)-5,5-bis(hydroxymethyl)-1,3-dioxane. They can be conveniently obtained as is exemplified in "Organic Synthesis", Collective Volume IV, p. 679 (1963) for the preparation of monobenzalpentaerythritol (named herein: 2-phenyl-5,5-bis(hydroxymethyl)-1,3-dioxane) by the reaction of pentaerythritol with an aldehyde or ketone under mildy acid conditions according to the equation:

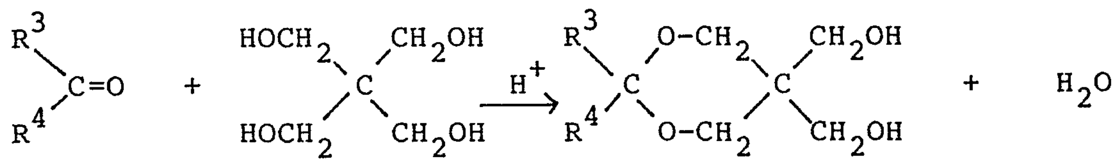

wherein $R^3$ and $R^4$ are as defined above.

Aldehydes and ketones that may be used in preparing the 1,3-dioxanes that can be used in Step a) of the preparation of the 2,2-bis(hydroxymethyl)-1,3-propanediol esters include any aldehyde or ketone not having functional groups other than the carbonyl group that is reactive with hydroxyl groups or that stearically hinders the carboxyl group from reacting with the hydroxyl group of pentaerythritol. Benzaldehyde and substituted benzaldehydes such as tolualdehyde, anisaldehyde and 4-dimethylaminobenzaldehyde are preferred.

Esterification of the mono and diorganic group substituted 5,5-bis(hydroxymethyl)-1,3-dioxanes (Compound III) is accomplished following conventional procedures by heating it together with a carbonyl compound, $R^2\overset{\overset{O}{\|}}{C}X$, (compound IV) in which $R^2$ and X are as defined above, preferably in the presence of a catalyst or base when X is halide and a polymerization inhibitor in accordance with the equation

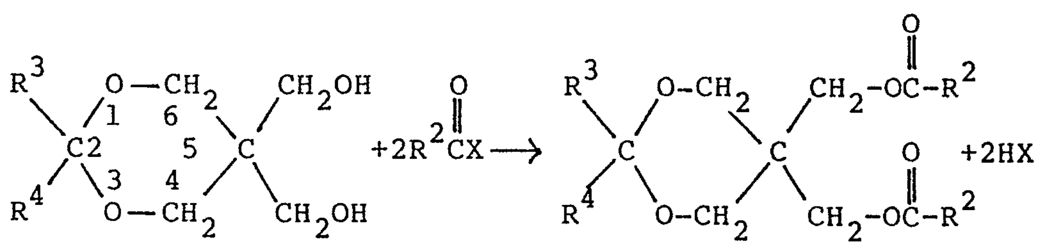

III IV V

mono or diorganic group substituted 5,5-bis(hydroxymethyl)-1,3-dioxane

mono or diorganic group-substituted 5,5-bis(hydroxymethyl)-1,3-dioxane esters

wherein $R^2$, $R^3$, $R^4$ and X are as defined above.

Carbonyl compounds, $R^2\overset{\overset{O}{\|}}{C}X$, that may be used in the esterification include any monovalent saturated or unsaturated aliphatic linear, branched, or cyclic group of 1 to 24 carbon atom-containing carboxylic acid, acid halide, anhydride, or ester of an alcohol containing 1 to 4 carbon atoms. The carbonyl compound may also be substituted by chlorine or fluorine. Examples of such carbonyl compounds are acrylic acid, methacrylic acid, crotonic acid, benzoic acid, cyclohexanecarboxylic acid, cyclohexenecarboxylic acid, butyric acid, heptafluorobutyric acid, perfluorooctanoic acid, stearic acid, and tetraeicosanoic acid and their acid chlorides, anhydrides and esters with methyl, ethyl, isopropyl, and butyl alcohols.

Hydrolysis of the mono or diorganic group-substituted 5,5-bis(hydroxymethyl)-1,3-dioxane esters is accomplished in accordance with the equation:

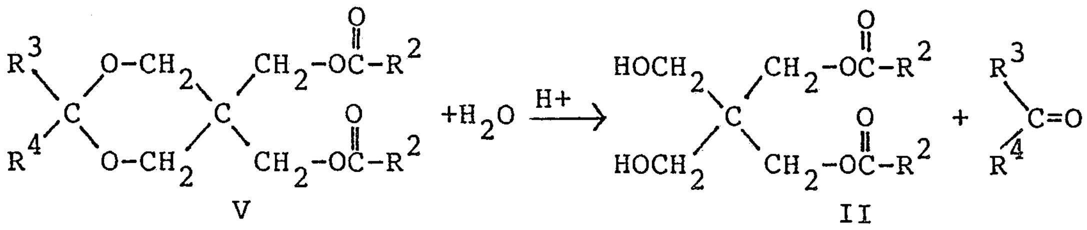

by following conventional procedures. Preferably, hydrolysis is accomplished by heating, at 20° to 100° C, an agitated mixture of the Compound V in water buffered to a pH about 2 to 5 for from 0.5 to 10 hours. Most preferably, the hydrolysis is accomplished by heating a rapidly agitated mixture of Compound V and an aqueous solution buffered at a pH of about one, e.g., a solution of potassium hydrogen oxalate and oxalic acid, and containing a polymerization inhibitor such as hydroquinone while steam distilling the by-product aldehyde or ketone. After the distillation of aldehyde or ketone is complete, the reaction mixture is cooled and the 2,2-bis(hydroxymethy)-1,3-propanediol esters extracted with a suitable solvent such as, for example, chloroform or ethyl acetate. The extract is dried over a drying agent or by azeotropic distillation of the water-solvent mixture and the esters isolated by distillation of the solvent, preferably under reduced pressure and in the presence of polymerization inhibitor.

The 2,2-bis(hydroxymethyl)-1,3-propanediol ester, Compound II, is a viscous hygroscopic liquid when $R^2$ is $-CH=CH_2$ (the diacrylate), a solid melting at 50° C when $R^2$ is $-C(CH_3)=CH_2$ (the dimethacrylate) and a liquid to semisolid when $R^2$ is a mixture of monovalent saturated and unsaturated aliphatic groups.

The 2,2-bis(hydroxymethyl)-1,3-propanediol esters, Compounds II, of the invention can be polymerized (see Steps c and d in the FLOW CHART, above) by methods known for polymerizing acrylic type monomers following bulk, solution, suspension, or emulsion techniques employing conventional catalysts, e.g., free-radical initiators. Where $R_1$ of Compound I is also an ethylenically-unsaturated group, i.e., $-CR=CH_2$ wherein R is hydrogen or methyl, highly hydrophilic crosslinked polymers are obtained. The esters can also be copolymerized with one or more ethylenically- unsaturated monomers to yield a great variety of hydroxyl group containing polymers. Examples of such comonomers that can be used are the alkyl esters of acrylic or methacrylic acid such as methyl methacrylate, ethyl acrylate, octyl acrylate, and benzyl acrylate, acrylamides such as methacrylamide; hydroxyalkylesters of acrylic and methacrylic acid such as 2-hydroxy ethyl acrylate and methacrylate, 2-hydroxy propyl acrylate and methacrylate; vinyl monomers such as vinyl chloride and vinyl acetate; vinylidene chloride; acrylonitrile; allyl compounds such as diallyl adipate; vinyl aryl compounds such as styrene and divinyl benzene; and other unsaturated compounds like butadiene and maleic anhydrive. In the copolymers, at least 1 mol percent, preferably 50 to 100 mol percent, more preferably 75 to 100 mol percent, are the 2,2-bis-(hydroxymethyl)-1,3-propanediol esters of the invention.

Suitable dicarboxylic acids that can be used in the preparation of the crosslinkable polyesters are saturated or unsaturated aliphatic, cycloaliphatic, or aromatic and can be substituted optionally by non-interferring groups such as alkyl, ester, halogen, and amido groups and include: succinic, glutaric, adipic, suberic, sebacic, undecanedicarboxylic, hexadecanedicarboxylic, dimerized fatty acids (such as obtained by the dimerization of olefinically unsaturated monocarboxylic acids containing 16 to 20 carbon atoms such as oleic acid, linoleic acid and the like). Other useful dicarboxylic acids are diglycolic, dilactic, 3,3′-(ethylenedioxy)dipropionic, phthalic, isophthalic, terephthalic, 5-sulfoisophthalic, diphenic, maleic, fumaric, itaconic, phenylenediacetic, benzylsuccinic, 1,4-naphthalenedicarboxylic, 5-chloro-1,3-benzene-dicarboxylic, tetrachlorophthalic, 1,2-cyclohexanedicarboxylic, 2,5-tetrahydrofuranedicarboxylic, 1,5-pent-2-enedioic, 2-methyleneglutaric, 2-methylenadipic, 3-methylitaconic, 3,3-dimethylitaconic and mixtures thereof.

Suitable diols that can be used in the preparation of the polyesters of the invention in addition to the required 2,2-bis(hydroxymethyl)-1,3-propanediol esters have a molecular weight of 62 to about 15,000 and include monomeric and polymeric diols. Examples of monomeric diols include straight or branched chain alkylenediols having the formula $HO(CH_2)_eOH$ in which e is 2 to 10 and oxaalkylenediols having a formula $H(OR^6)_fOH$ in which $R^6$ is an alkylene group having 2 to 4 carbon atoms and f is 2 to 4. Examples include ethyleneglycol, propyleneglycol 1,4-butanediol, 1,6-hexanediol, 2,2-dimethyl-1,3-propanediol, diethyleneglycol, 1,11-(3,6-dioxa)undecanediol and the like. Examples of polymeric diols include poly(oxyalkylene)diols such as the Carbowax® diols available from Union Carbide, the poly(oxytetramethylene) diols such as Polymeg® diols available from Quaker Oats Company and the Terethane® diols available from duPont, the polyester diols such as Multron® poly(ethylene adipate)diols available from Mobay Chemical Co., the polycaprolactone diols such as the "Tone"® diols available from Union Carbide, the polyolefin diols such as the polyethylenediols available from Arco Chemical Co. and the polysiloxanediols such as those described in U.S. Patent No. 3,886,865 but preferably those described in U.S. Patent No. 4,013,698.

In preparing the polyesters, the diols of which at least 1 mol percent, but preferably 75 to 100 mol percent, are the 2,2-bis(hydroxymethyl)-1,3-propanediol esters of the invention and the dicarboxylic acids are

subjected to polycondensation procedures which are well known in the art. That is, the diols and dicarboxylic acid halide or anhydride are mixed in an agitated tank or thin film reactor with an appropriate catalyst and/or a co-reagent base and optionally a stabilizing agent and heated to reaction temperature, generally to about 50 to 100°C, for about one to ten hours. Generally, the diol and dicarboxylic acids or derivatives are employed in stoichiometric proportions although either component can be employed in an excess of up to about 100% by weight but preferably an excess of up to about 5% of either component is used. Also, usually an inhibitor for the prevention of the polymerization of the acrylic groups during the polycondensation reaction is desirable. Examples of inhibitors are hydroquinone 4-methoxyphenol, and phenothiazine.

Suitable diisocyanate that can be used in the preparation of the crosslinkable polyurethane are well known and include any of the linear, branched, and cyclic aliphatic, aromatic and heterocyclic diisocyanates known in the polyurethane field. Examples of preferred diisocyanates include 2,4-tolylene diisocyanate, 3,5,5-trimethyl-1-isocyanato-3-isocyanatomethylcyclohexane, trimethylhexamethylenediisocyanate, m-tetramethylxylylenediisocyanate, p-tetramethylxylylenediisocyanate, methylene-bis(4-cyclohexylisocyanate), hexamethylene diisocyanate, and 1,3-di(isocyanatoethyl)hydantoin.

In preparing the polyurethanes, the diols of which at least 5 mol percent, but preferably, 50 to 100 mol percent, more preferably 75 to 100 mol percent, are the 2,2-bis(hydroxymethyl)-1,3-propanediol esters of the invention and the diisocyanates are mixed together at temperatures such that a melt is obtained. Generally, a temperature of 20 to 125°C depending on the liquid or solid state of the reactants. The reaction can be carried out in an aprotic non-polar or polar solvent, for example, toluene, acetone, methyl ethyl ketone, ethyl acetate, or tetrahydrofuran. It is also desirable to use a catalyst for the reaction such as, for example, dibutyltin laurate or a metal chelate such as iron tris(acetylacetonate). Where temperatures higher than about 50°C are used for the reactions, it may be advantageous to add a polymerization inhibitor such as 4-methoxyphenol. The solvent, when used, can be removed by evaporation, distillation, or freeze drying or the polyurethane can be precipitated by addition of a nonsolvent for the polymer, e.g., diethyl ether or hexane and filtered.

The polyesters and polyurethanes of the invention are thermoplastic and because of the pendent acrylic or methacrylic groups articles of various shapes can be molded from compositions containing them and crosslinked to become insoluble and infusible. Generally, a heat activated free-radical initiator is incorporated into the polyester or polyurethane compositions in an amount from about 0.1 to 5.0 percent by weight of total weight of the composition. Examples of heat activated free-radical initiators include but are not limited to benzoyl peroxide, lauroyl peroxide, dicyclohexyl percarbonate, azo-bis(isobutyronitrile) and the like. The free-radical initiated molded article can then be crosslinked by heating, preferably at a temperature between 50 and 150°C. Radiation activated free-radical initiators can be used in the composition, however, they are not preferred when the molded article has a thickness greater than about 5 millimeters.

The polyesters and polyurethanes of the invention are soluble in many organic solvents, e.g. acetone, methyl ethyl ketone, tetrahydrofuran, propyl acetate, from which thermoplastic coatings can be cast and cured to solvent, heat and abrasion resistant coatings. Curing of the coatings may be effective by heat or by exposure to actinic radiation. Generally, compositions suitable for coating comprise a solution of from about 5 to 50, preferably 10 to 25 percent by weight of the polyester or polyurethane in a suitable solvent and a heat or a radiation activated polymerization initiator.

Heat and radiation activators of polymerization are well known. The preferred initiators are the radiation activated initiators. Included among such initiators are acyloin and derivatives thereof, such as benzoin, benzoin methyl ether, benzoin ethyl ether, benzoin ispropyl ether, benzoin isobutyl ether, and 2-hydroxy-2-methyl-1,2-diphenylethanone; diketones such as benzil and diacetyl; phenones such acetophenone, 2,2,2-tribromo-1-phenylethanone, 2,2-diethoxyacetophenone, 2,2-dimethoxy-2-phenylacetophenone, 2,2,2-tribromo-1-(2-nitrophenyl)ethanone, benzophenone, 4,4′-bis(dimethylamino)benzophenone and 1-hydroxycyclohexyl phenyl ketone. Normally, the initiator is used in amounts ranging from about 0.01 to 10% by weight of the total polymerization composition comprising polyester or the polyurethane. When the quantity is less than 0.01% by weight, the polymerization rate becomes extremely low. If the initiator is used in excess of about 10% by weight, no correspondingly improved affect can be expected. Thus, addition of such greater quantity is not economically justified and may distract from the properties of the cured coatings. Preferably, about 0.25 to 5% of initiator is used in the polymerizable composition.

The photopolymerization of the compositions of the invention occurs on exposure of the compositions to any source of radiation emitting actinic radiation at a wavelength within the ultraviolet and visible spectral regions and by infrared radiation, i.e. thermal energy. Suitable sources of radiation include mercury, xenon, carbon arc and tungsten filament lamps, sunlight. Exposures may be from less than about one second to ten minutes or more depending upon the amounts of particular polymerizable materials, the photopolymerization catalyst being utilized, the radiation source, the distance of the composition from the source and the thickness of the coating to be cured. The compositions may also be polymerized by exposure to electron beam irradiation. Generally speaking, the dosage necessary is from less than 1 megarad to 30 megarads or more. An advantage of curing with electron beam irradiation is that highly pigmented compositions can be effectively cured at a faster rate than by mere exposure to actinic radiation.

In addition to solvents, catalysts, and initiators of polymerization, the polyester and polyurethane compositions to be cured can also include other materials such as dyes; pigments such as titanium dioxide, clay, silica, calcium carbonate, and zinc chromate; fillers such as glass and carbon fibers, glass beads and bubbles, organic polymer fibers and particles. These additives may be present in quantities up to 500 parts or

more per 100 parts polyester or polyurethane by weight and preferably from about 0.01 to about 200 parts on the same basis.

The esters of the invention which are uncontaminated by monools, triols, and polyols are useful as abrasion-resistant coatings for metals, plastics, and particularly wooden articles, especially furniture.

Objects and advantages of this invention are further illustrated by the following examples, but the particular materials and amounts thereof recited in these examples, as well as other conditions and details, should not be construed to unduly limit this invention.

EXAMPLE 1 - preparation of 2,2-bis(acryloyloxymethyl)-1,3 propanediol

Step a) esterification of 2-phenyl-5,5-bis(hydroxymethyl)-1,3-dioxane

Into a two-liter reaction flask equipped with an agitator, reflux condenser, and dropping funnel was placed one liter of dichloromethane, 224g (1.0 mole) of 2-phenyl-5,5-bis(hydroxymethyl)-1,3-dioxane and 222 g (2.2 moles) triethylamine. Into the flask was added dropwise 190 g (2.1 moles) of acryloyl chloride at a rate sufficient to maintain gentle reflux. After the addition was complete, the mixture was stirred for 30 minutes and allowed to cool. The flask was then immersed in ice-water and stirring continued until the flask contents reached a temperature of 5° C or less. Triethylammonium chloride was filtered from the reaction mixture and the filter cake rinsed with cold dichlormethane. The combined filtrates were washed with cold water and a saturated solution of sodium sulfate. The washed filtrates were dried over anhydrous sodium sulfate and then filtered through silica gel. The solvent was then removed by evaporation under reduced pressure and the crude product, 2-phenyl-5,5-bis (acryloyloxymethyl)-1,3-dioxane, used in Step b directly or after recrystallization from ethyl acetate or diethyl ether (m.p. 65° C).

The 2-phenyl-5,5-bis(hydroxymethyl)-1,3-dioxane used in Step a) was obtained by the reaction of benzaldehyde and pentaerythritol according to the procedure described in Org. Synthesis, Col. Vol. p. 679 (1963).

Steb b) - hydrolysis of 2-phenyl-5,5-bis(acryloyloxymethyl)-1,3-dioxane

Into a two-liter flask equipped with an agitator, heating bath, means for steam distillation, and a Claisen head with an efficient condenser was placed 500g (1.5 moles) of 2-phenyl-5,5-bis(acryloyloxymethyl)-1,3-dioxane, two grams of 4-methoxyphenol and a hot solution of 4g of oxalic acid and 0.8g of potassium carbonate in 100ml of water. A slow stream of air was introduced into the steam inlet and the mixture stirred and warmed until the mixture appeared to be a homogeneous emulsion. Steam was then added to the air stream whereon benzaldehyde began to steam distill. It is desirable during the steam distillation that rapid stirring be maintained to provide intimate contact between the organic and aqueous layers in order to decrease the time required for hydrolysis as much as possible. After benzaldehyde was no longer distilling, the still residue was cooled, filtered, saturated with sodium sulfate, and extracted with ethyl acetate. The combined extracts were dried, preferably using anhydrous sodium sulfate followed by azeotropic distillation of water and ethyl acetate under reduced pressure. The 2,2-bis-(acryloyloxymethyl)-1,3-propanediol obtained was a viscous, hygroscopic liquid. NMR spectroscopy and gas chromotography confirmed the fact that no monol or triol was present in the product.

Similar results were obtained by repeating the procedure of Example 1 using in place of 2-phenyl-5,5-bis(hydroxymethyl)-1,3-dioxane, 2,2-dimethyl-5,5-bis(hydroxymethyl)-1,3-dioxone (from acetone), or 2-(4-methoxyphenyl)-5,5-bis(hydroxymethyl)-1,3-dioxane (from anisaldehyde).

EXAMPLE 2 - preparation of 2,2-bis(methacryloyloxymethyl)-1,3-propanediol

Step a) esterification of 2-(4-methoxyphenyl)-5,5-bis-(hydroxymethyl)-1,3-dioxane

The procedure of Example 1 Step a) was followed using in place of 2-phenyl-5,5-bis(hydroxymethyl)-1,3-dioxane 254g (1.0 mol) of 2-(4-methoxyphenyl)-5,5-bis(hydroxymethyl)-1,3-dioxane and in place of acryloyl chloride 219.5 (2.1 moles) methacryloyl chloride. There was obtained 2-(4-methoxyphenyl)- 5,5-bis(methacryloyloxymethyl)-1,3-dioxane having a melting point of 114-116° C after crystallation from ethyl acetate.

Step b) hydrolysis of 2-(4-methoxyphenyl)-5,5-bis (methacryloyloxymethyl)-1,3-dioxane

The procedure of Example I Step b) was followed using in place of 2-phenyl-5,5-bis(acryloyloxymethyl)-1,3-dioxane 500g of 2-(4-methoxyphenyl)-5,5-bis(methacryloyloxymethyl)-1,3-dioxane. There was obtained 2,2-bis(methacryloyloxymethyl)-1,3-propanediol having a melting point of about 50° C when recrystallized from ethyl acetate. NMR spectroscopy and gas chromatography confirmed the fact that no monol or triol was present in the product.

Similar results were obtained by repeating the procedure of Example 2 using in Step a) either 2-phenyl-5, 5-bis(hydroxymethyl)-1,3-dioxane, 2,2-dimethyl-5,5-bis(hydroxymethyl)-1,3-dioxane, 2-(4-methylphenyl-5,5-bis(hydroxymethyl)-1,3-dioxane or 2-(4-chlorophenyl-5,5-bis(hydroxymethyl)-1,3-dioxane in place of 2-(4-methoxyphenyl)-1,3-dioxane.

EXAMPLE 3 - preparation of 2,2-bis(hydroxymethyl)-1,3-propanediol mixed ester

The procedure of Example 1 was followed with the exception that in the esterification step a mixture of 95 g of acryloyl chloride (1.05 mole) and 82.4 g of acetyl chloride (1.05 mole) was used in placed of 2.1 moles of acryloyl ohloride. The product obtained was semicrystalline mixture consisting of approximately a 1:1:2 mole distribution of 2,2-bis(acryloyloxymethyl)-1,3-propanediol, 2,2-bis(acetyloxymethyl)-1,3-propanediol, and 2-acryloyloxymethyl-2-acetyloxymethyl-1,3-propanediol.

In a similar manner mixtures of 2,2-bis(hydroxymethyl)-1,3-propanediol acrylate-benzoate and acrylate-stearate were prepared using mixtures of acryloyl chloride with either benzoyl chloride or stearyl chloride in place of acryloyl chloride.

EXAMPLE 4 - preparation of the solvent soluble, crosslinkable polyurethane having pendent acrylic ester groups

Into a 500 ml flask equipped with a mechanical stirrer, condenser with a drying tube, and means for introducing a stream of gas below the surface of a fluid in the flask was placed 122 g (0.5 mole) 2,2-bis(acryloyloxymethyl)-1,3-propanediol (that had been dried by azeotropic distillation of ethyl acetate and inhibited by methyl hydroquinone), 230 ml of ethyl acetate, and 111g isophorone diisocyanate, IPDI. There was then added 0.1 ml of dibutyltin dilaurate and a slow stream of air passed through the solution. The solution was stirred and a heating bath at 75°C applied to the flask. These conditions were maintained for 70 hours during which time isocyanate content of the flask was monitored by infrared spectroscopy. At the end of this time, isocyanate had disappeared. The reaction mixture was poured while stirring rapidly into 500 ml of ether. The white fibrous material obtained was found to have an inherent viscosity of 0.11 and an acrylic ester equivalent weight of 230. The polymer was readily soluble in ethyl acetate, methyl ethyl ketone, acetone, ethanol, and other common solvents.

A solution containing 30% by weight of the polymer was prepared and 2% by weight based on dissolved solids of the photoinitiator Irgacure® 184 (Ciba-Geigy) added. The solution was coated using a Meyer bar at a dried thickness of about 25 micrometers on 100 micrometer polyester film and air dried over night. A portion of the dried coating was cured in air and another under nitrogen in RPC® UV Processor (Radiation Processing Inc., subsidiary of Sumitomo Heavy Industries Ltd., Tokyo, Japan) having two lamps set on high (about 120 watts/cm.) using two passes at a belt speed of 25 centimeters per second. Abrasion resistance of each was measured by abrading on a Taber Abraser® available from Pacific Scientific Co. using 30 cycles with CS-17 wheels under a 500g load and quantified as percent haze (average of several samples) with a Hazegard® hazemeter available from Pacific Scientific Co. The sample cured in air showed a haze of 8.2% and the sample cured under nitrogen showed a haze of 4.5%.

EXAMPLES 5-10

Various crosslinkable polyurethanes of the invention were prepared and evaluated according to the procedures followed in Example 4 using the mole equivalents of 2,2-bis(hydroxymethyl)-1,3-propanediol diacrylate (the diol), the diisocyanate, and, where used, the additive given in Table I. In Table I is also shown the percent haze for each cured polyurethane.

The data of Table I show that in most cases the polymers of the invention provide highly abrasive resistant coatings.

TABLE I

| Ex. No. | Moles diol | Diisocyanate (moles) | Additive (moles) | IV[i] | Percent haze In Air 2%[j] | Percent haze In Air 4%[j] | Percent haze In Nitrogen 2%[j] | Percent haze In Nitrogen 4%[j] |
|---|---|---|---|---|---|---|---|---|
| 5 | 1 | [a]TMDI (1) | -- | 0.09 | 3.2 | 2.9 | 2.5 | 2.1 |
| 6 | 1 | [b]TMXDI (1) | -- | | 9.1 | 7.8 | | |
| 7 | 3 | TMDI (4) | [f]HEA (2) | 0.05 | 3.9 | 3.0 | 2.4 | 2.5 |
| 8 | 3 | [c]H12MDI (4) | HEA (2) | 0.09 | 2.9 | 2.7 | | |
| 9 | 1 | [d]HDI (2) | [g]NPG (1) | 0.41 | 3.2 | 3.4 | | |
| 10 | 4 | [e]TDI (5) | [h]PCD (1) | 0.19 | 3.9 | 3.9 | | |

[a]2,2,4- and 2,4,4-trimethyl-1,6-hexane diisocyanate
[b]tetramethyl-m-xylene diisocyanate
[c]dicyclohexylmethane diisocyanate
[d]hexane diisocyanate
[e]tolylene diisocyanate
[f]hydroxyethyl acrylate
[g]neopentyl glycol
[h]polycaprolactone diol (MW 830), from Union Carbide as Tone 210™
[i]inherent viscosity measured on 1% w/v solutions in 50/50 v/v methyl ethyl ketone/ethanol
[j]weight percent of the photoinitiator Irgacure 184

Various modifications and alterations of this invention will become apparent to those skilled in the art without departing from the scope and spirit of this invention, and it should be understood that this invention is not to be unduly limited to the illustrative embodiments set forth herein.

## Claims

1. A 2,2-bis(hydroxymethyl)-1,3-propanediol ester of at least one of acrylic and methacrylic acids uncontaminated by monools, triols, and higher hydroxyl group-containing oligomers of pentaerythritol.

2. The ester according to claim 1 uncontaminated by monools, triols, and higher hydroxyl group containing oligomers of pentaerythritol having the formula:

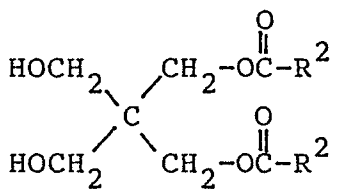

wherein each $R^2$ is the same or different monovalent saturated or unsaturated aliphatic, branched, or cyclic groups having 1 to 24 carbon atoms or aromatic groups having 6 to 18 carbon atoms, and wherein $R^2$ optionally is substituted by at least one group which is unreactive in the esterification process and wherien the $R^2$ group optionally further contains fluorine, chlorine, or ether oxygen, with the proviso that at least 1 mole percent by weight of the $R^2$ groups are $-\underset{R}{\underset{|}{C}}=CH_2$,
in which R is hydrogen or methyl.

3. The ester according to claims 1 and 2 having the formula:

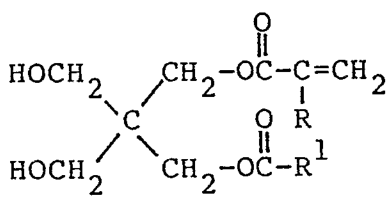

wherein
R is hydrogen or methyl; and
$R_1$ is a saturated or unsaturated aliphatic linear, branched, or cyclic group having 1 to 24 carbon atoms, and wherein $R_1$ is optionally substituted by at least one chlorine or fluorine atom, or ether oxygen.

4. A process for preparing a 2,2-bis(hydroxymethyl)-1,3-propanediol ester of acrylic or methacrylic acid according to claims 1 to 3 or mixtures of these esters with at least one 2,2-bis(hydroxymethyl)-1,3-propanediol ester of other saturated and unsaturated aliphatic and aromatic monocarboxylic acids, the esters and mixtures of esters being uncontaminated by monools, triols, and higher hydroxy group-containing oligomers of pentaerythritol comprising the steps of:

(1) esterifying 2-mono or 2,2-diorganic group-substituted 5,5-bis(hydroxymethyl)-1,3-dioxane with a carbonyl compound having the formula:

$R^2\overset{O}{\overset{\|}{C}}X$, wherein $R^2$ is defined above, and X is hydroxyl, halogen, alkoxy having 1 to 4 carbon atoms, or $-O\overset{O}{\overset{\|}{C}}R^2$;

2) hydrolyzing the esterified dioxane compound; and

3) isolating the resulting ester.

5. The process according to claim 4 wherein said < dioxane is selected from the group consisting
2,2-dimethyl-5,5-bis(hydroxymethyl)-1,3-dioxane,
2,2-diethyl-5,5-bis(hydroxymethyl)-1,3-dioxane,
2-methyl-2-ethyl-5,5-bis(hydroxymethyl)-1,3-dioxane,
2-phenyl-5,5-bis(hydroxymethyl)-1,3-dioxane,

2-(4-dimethylaminophenyl)-5,5-bis(hydroxymethyl)-1,3-dioxane and
2-(4-methoxyphenyl)-5,5-bis(hydroxymethyl)-1,3-dioxane.

6. The process according to claims 4 and 5 wherein $R^2 \overset{\overset{O}{\|}}{C} X$, is a carboxylic acid, acid halide, anydride, or ester of a $C_1$ to $C_4$ alcohol and wherein the $R^2$ group is a monovalent aliphatic linear, branched, or cyclic group having 1 to 24 carbon atoms.

7. The process according to claim 10 wherein said $R^2 \overset{\overset{O}{\|}}{C} X$ compound is substituted by at least one fluorine or chlorine atom.

8. The process according to claims 4 to 7 wherein said carbonyl compound is selected from the group consisting of acrylic acid, methacrylic acid, crotonic, acid, acetic acid, propionic acid, benzoic acid, cinnamic acid, cyclohexanecarboxylic acid, cyclohexenecarboxylic acid, butyric acid, heptafluorobutyric acid, perfluorooctanoic acid, stearic acid, tetraeicosanoic acid, and their acid chlorides, anhydrides and esters with methyl, ethyl, propyl, isopropyl, butyl sec-butyl and tert-butyl alcohols.

9. The process according to claims 4 to 8 wherein said hydrolyzing step takes place at a temperature in the range of 20 to 100° C in an aqueous solution having a pH in the range of 0.5 to 5.

10. The process according to claims 4 to 9 wherein said hydrolyzing step takes place in the presence of a polymerization inhibitor.